# DEMANDE DE BREVET EUROPEEN

(11) **EP 1 741 459 A1**
(43) Date de publication de la demande: **10.01.2007**
(21) Numéro de dépôt: 05405428.3
(22) Date de dépôt: 08.07.2005
(51) Int. Cl.: A61M 5/32, B29C 45/00

(54) **Dispositif multifonctionnel pour aiguilles de seringue**

(71) Demandeur: Biofluid Systems S.A., 1260 Nyon (CH)
(72) Inventeur: Rochat, Jean-Denis, 1272 Genolier (CH); Marti, René-Andréas, 4153 Reinach (CH)
(74) Mandataire: Savoye, Jean-Paul

(57) **Abrégé**

Dispositif (1) multifonctionnel pour aiguilles (30) de seringues comprenant au moins un premier logement (10), abritant au moins une aiguille (30) destinée à en être extraite par un embout (31) constitutif de cette aiguille, et autant de seconds logements (20) qu'il y a d'aiguilles (30) pour piéger ces dernières en les introduisant chacune dans un second logement (20). Au moins le premier logement (10) et ladite aiguille (30), pourvue de son embout (31), forment un ensemble moulé d'un seul tenant.

## Description

La présente invention a pour objet un dispositif multifonctionnel pour aiguilles de seringue permettant d'une part de garder cette aiguille, d'abord stérile, à l'abri des personnes manipulant le dispositif, et d'autre part de fournir un logement clos duquel cette aiguille ne sera plus ressortie une fois usagée.

Un tel dispositif trouve son application dans le domaine médical et peut être destiné en particulier à l'usage des anesthésistes durant une opération.

Il existe plusieurs dispositifs de ce genre parmi lesquels on citera celui décrit dans le document WO 97/40869 qui concerne un capuchon de sécurité pour aiguille de seringue. L'aiguille et le capuchon de sécurité sont conditionnés en milieu stérile par un même emballage que l'on ouvrira avant de pouvoir faire usage de ces deux éléments qui constituent son contenu.

Le capuchon de sécurité décrit dans ce document antérieur est formé d'un corps en plastique comprenant un premier compartiment dans lequel est fixée une aiguille métallique. Cette aiguille est terminée d'un embout permettant de la connecter au corps d'une seringue par une connexion de type luer ou luer-lock bien connue dans le domaine. De forme conique, cet embout comprend une pluralité de rainures radiales destinées à s'engager dans des saillies solidaires du premier compartiment. L'emboîtement des rainures dans ces saillies permet le maintien de l'aiguille dans son compartiment. Juxtaposé à ce dernier, se trouve un second compartiment destiné à piéger l'aiguille après en avoir fait usage. Pour ce faire, ce second compartiment comprend une ouverture pourvue d'un collet flexible venant en prise avec l'embout de l'aiguille. Une fois celle-ci insérée, ce collet empêche tout retrait de l'aiguille, laquelle est alors piégée dans un compartiment clos de sécurité.

Le document US 5'718'689 décrit un autre dispositif semblable au précédent mais qui diffère toutefois de ce dernier, essentiellement par le moyen utilisé pour piéger l'aiguille.

Les deux dispositifs précédents sont chacun dotés d'une assise leur permettant de se maintenir d'eux-mêmes debout sur une surface plane. Ainsi, il n'est plus nécessaire de devoir tenir d'une main ce dispositif pour retirer ou insérer de l'autre l'aiguille au moyen du corps de la seringue. Afin d'assurer une bonne stabilité de l'objet, ces assises sont formées d'une surface plane relativement étendue et si nécessaire revêtue d'un adhésif. Un des modèles prévoit notamment qu'une telle assise puisse être escamotée afin de pouvoir bénéficier d'une grande surface qui ne soit pour autant pas encombrante lorsque le dispositif n'est pas ou plus utilisé. Enfin, les deux dispositifs prévoient également que leurs compartiments puissent être réalisés en une seule pièce de plastique par un procédé de moulage par injection.

L'un des inconvénients de ces dispositifs réside dans le fait qu'ils présentent un coût de fabrication relativement élevé pour un objet à usage unique, à savoir un objet devant être jeté après sa première utilisation. En effet, la réalisation de ces objets nécessite dans une première étape la fabrication de l'aiguille métallique, puis dans une seconde étape la fabrication du boîtier de cette aiguille constitué des deux compartiments, et dans une troisième étape l'assemblage de l'aiguille à son boîtier dans des conditions stériles avant l'emballage du tout.

Un autre inconvénient découle du fait que le moyen utilisé pour le maintien de ces dispositifs en position adéquate ne permet pas de les fixer à un montant d'un statif de perfusion par exemple, mais nécessite le recours à une surface plane d'une dimension suffisante.

Le but de la présente invention vise à remédier au moins en partie aux inconvénients précités en suggérant un dispositif multifonctionnel conforme à ce qu'énonce la revendication 1.

Le principal avantage de ce dispositif réside dans le fait qu'il soit simple et économique à réaliser. En outre, il présente également l'avantage de pouvoir constituer un élément unitaire qui, assemblé à d'autres éléments semblables ou identiques, permet de constituer très simplement un support à seringues d'un seul bloc, parfaitement adapté aux besoins de chaque intervention médicale.

D'autres avantages apparaîtront à la lumière de la description qui va suivre et qui se réfère à un mode de réalisation préféré, pris à titre nullement limitatif, et illustré par les figures annexées dans lesquelles:
La figure 1 est une vue schématique en perspective du dispositif de la présente invention, dans un état initial correspondant à celui où l'aiguille se trouve maintenue dans son premier logement.
La figure 2 est une vue de dessus de la figure 1.
La figure 3 est une vue schématique en perspective de l'aiguille sortie de son premier logement.
La figure 4 est une vue schématique en perspective montrant la partie postérieure d'une partie du dispositif dans un second état, à savoir celui où l'aiguille a été retirée de son premier logement.
La figure 5 est une vue schématique semblable à celle de la figure 1, montrant le dispositif dans un troisième état, à savoir celui où l'aiguille a été insérée dans son second logement.
La figure 6 donne, dans une vue schématique en perspective, une illustration d'un premier support pour le dispositif de la présente invention.
La figure 7 illustre, dans une vue schématique en perspective, une variante du dispositif 1.
La figure 8 représente, en variante, un autre support pour la variante du dispositif 1 illustré à la figure 7.

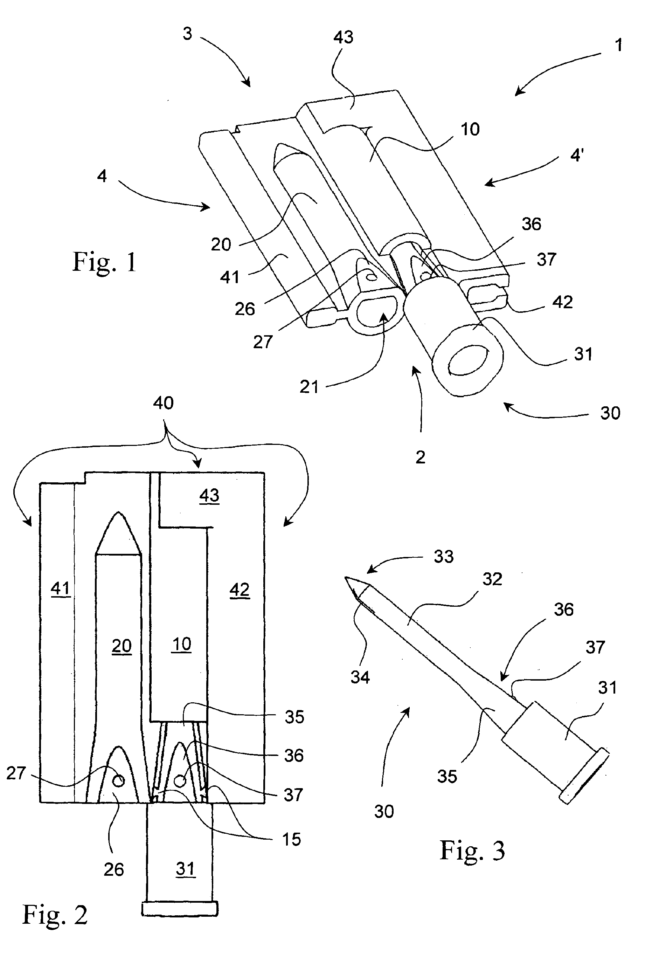

En référence à la figure 1, le dispositif 1 de la présente invention est illustré dans une vue schématique en perspective plongeante depuis sa partie frontale 2. L'extrémité postérieure 3 de ce dispositif sera définie comme étant celle opposée à la partie frontale 2 et les extrémités latérales 4, 4' comme étant celles situées respectivement à gauche et à droite du dispositif tel que représenté dans cette figure.

Le dispositif 1 comprend au moins un premier logement 10 abritant au moins une aiguille 30 destinée à en être extraite, par un embout 31 constitutif de cette aiguille, au travers d'une première ouverture 11. Cette aiguille est représentée seule dans la figure 3 selon une vue schématique en perspective. Elle se compose d'une partie effilée 32 creuse, terminée en l'une de ses extrémités par une pointe 33 et une fente 34 nécessaire au passage du liquide devant remplir le corps non représenté d'une seringue. L'autre extrémité de l'aiguille 30 comprend une portion conique 35 permettant d'une part de relier la partie effilée 32 à l'embout 31, et d'autre part d'améliorer la rigidité de l'aiguille. Comme mieux visible sur les figures 1 et 2, cette portion conique est en partie tronquée pour former un replat 36 sur lequel se situe une saillie 37 ayant de préférence la forme d'une petite demi-sphère.

L'embout 31 est un embout de type luer ou luer-lock autorisant la connexion de l'aiguille 30 au corps de la seringue. Ce type de connexion est formé de cônes normalisés bien connus de l'homme du métier. Les embouts luer-lock se différentient des embouts luer par le fait qu'ils comportent un pas de vis alors que les embouts luer s'assemblent par simple emboîtement dans l'embase du corps de l'aiguille.

Tel qu'illustrée aux figures 1 et 2, l'aiguille 30 est initialement située dans son premier logement 10. Ce dernier permet de couvrir, au moins partiellement, la partie effilée 32 de l'aiguille de façon à ce que le dispositif 1 puisse être manipulé sans toucher cette partie de l'aiguille qui doit rester stérile.

La figure 2 représente l'objet de la présente invention dans une vue de dessus. Dans cette figure, on remarque que l'aiguille est maintenue dans le premier logement 10 par un moyen de fixation 15 susceptible d'être brisé. De préférence ce moyen de fixation est constitué par au moins un cône de matière, deux dans le présent cas, dont la base est jointe au premier logement 10 et dont le sommet est joint à l'aiguille 30. Grâce au moyen de fixation 15, au moins le premier logement et l'aiguille pourvue de son embout forment un ensemble d'un seul tenant. Un tel ensemble de pièces sera obtenu par moulage d'une matière injectée dans un moule, notamment d'une matière plastique.

Selon le mode de réalisation préféré, les deux cônes formant le moyen de fixation 15 sont situés dans un même plan, passant par l'axe longitudinal de l'aiguille, et leur base se trouve rattachée à la paroi de la première ouverture 11 du premier logement 10. La forme générale de cette première ouverture est de préférence semblable à celle de la portion conique 35 de l'aiguille.

A coté du premier logement 10 se trouve un second logement 20 destiné à piéger l'aiguille après en avoir fait usage. Ce second logement sert également de moyen pour séparer l'aiguille du corps de la seringue. En effet, pour des opérations d'anesthésie par exemple, l'aiguille connectée au corps de la seringue ne sera utilisée que pour remplir cette dernière. Cette opération s'effectue en aspirant un produit, par exemple contenu dans une fiole ou dans un vial après percement de la membrane de ce dernier. Une fois la seringue remplie, l'aiguille devient inutile du fait que les injections se font au travers de cathéters disposant d'accès luer ou luer-lock. Il convient donc d'ôter cette aiguille sans risquer de se piquer. Le dispositif de la présente invention permet également de réaliser cette opération par le biais du second logement 20 qui va être décrit maintenant.

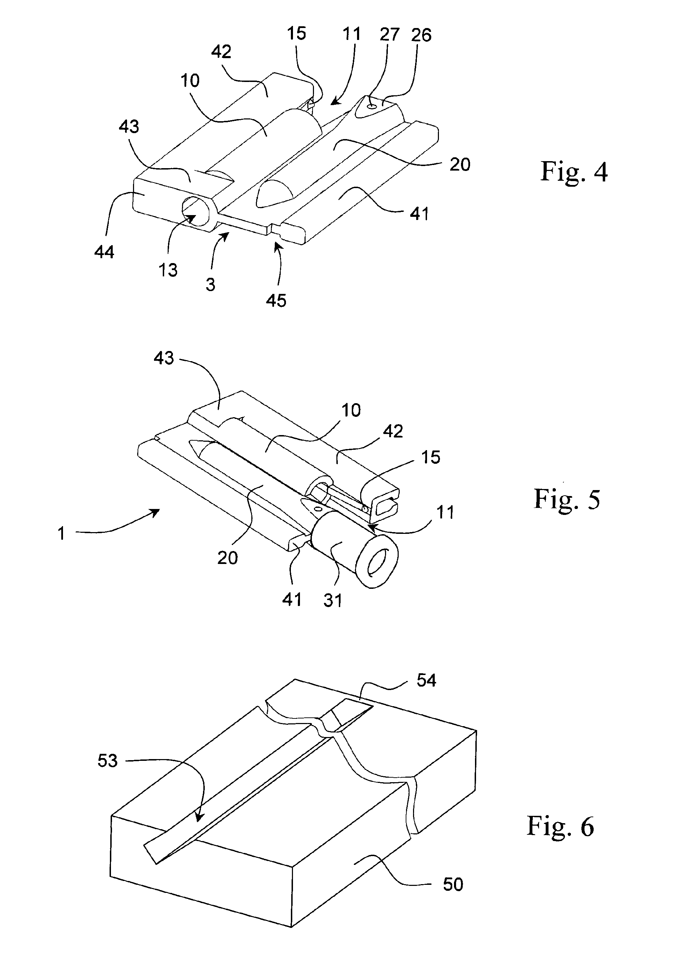

Le second logement 20 est un logement borgne présentant une seconde ouverture 21 ayant de préférence une forme identique, ou du moins complémentaire, à celle de la portion conique 35 de l'aiguille. De ce fait, la seconde ouverture 21 est préférentiellement conique et dispose également d'un plat 26 semblable ou identique au replat 36. La forme conique de cette seconde ouverture facilite l'introduction de l'aiguille dans son second logement. Le plat 26 est percé d'un trou 27 destiné à accueillir la saillie 37 de l'aiguille lorsque cette dernière est introduite dans le second logement, comme illustré à la figure 5.

Le plat 26, le trou 27 ainsi que le replat 36 et la saillie 37 constituent un moyen de blocage pour l'aiguille qui empêche tout mouvement de rotation ou de translation de celle-ci par rapport au second logement 20. L'aiguille étant bloquée dans tous ses degrés de liberté, le corps de la seringue peut alors être séparé de l'aiguille par un simple mouvement d'une seule main en toute sécurité. Après, utilisation de la seringue, celle-ci peut avantageusement être reconnectée à l'aiguille piégée dans son second logement. Ce dernier fait alors office de bouchon pour la seringue, garantissant ainsi la stérilité du produit qu'elle contient.

Avantageusement, le plat 26 de la seconde ouverture conique oblige le praticien à introduire l'aiguille 30 dans le second logement 21 selon une position déterminée facilement compréhensible. Cette position particulière correspondant à celle permettant de faire fonctionner le moyen de blocage de l'aiguille. En outre on notera que ce moyen de blocage pourrait être amélioré par un organe additionnel ou par un autre moyen empêchant définitivement tout retrait de l'aiguille hors de son second logement.

En référence à la figure 2, le dispositif 1 peut être avantageusement doté d'au moins un moyen d'assemblage 40, par emboîtement, autorisant sa connexion à un moyen d'assemblage complémentaire non représenté. Comme mieux visible dans les figures 1 et 5, ce moyen d'assemblage est pourvu d'au moins un coulisseau 41 et d'au moins une glissière 42, de préférence diamétralement opposée au coulisseau 41. Le coulisseau et la glissière sont préférentiellement disposés aux extrémités latérales 4, 4' du dispositif 1. Les formes du coulisseau et de la glissière sont complémentaires de sorte que le coulisseau 41 d'un premier dispositif 1 puisse être introduit dans la glissière 42 d'un second dispositif 1. Dans ce cas, la glissière 42 du second dispositif 1 constituerait le moyen d'assemblage complémentaire du coulisseau 41 du premier dispositif 1.

Selon le mode de réalisation préféré, la connexion constituée par l'emboîtement du premier moyen d'assemblage 40 dans le moyen d'assemblage complémentaire n'autorise qu'un déplacement linéaire de ces moyens l'un par rapport à l'autre une fois ceux-ci emboîtés. De ce fait, le coulisseau présentera une section essentiellement non circulaire, par exemple carrée ou rectangulaire tel que représenté dans les figures 1, 4 et 5. Cette caractéristique permet avantageusement d'éviter toute articulation de la connexion, contrairement au cas où le coulisseau présenterait une forme essentiellement cylindrique.

En référence à la figure 4, laquelle présente la partie postérieure d'une partie du dispositif 1, on remarque qu'une butée 44 permet de limiter le déplacement linéaire du moyen d'assemblage 40 par rapport à son moyen d'assemblage complémentaire. Selon l'illustration donnée dans cette figure, le déplacement linéaire du coulisseau 41 par rapport à la glissière 42 se trouve donc limité du côté de la butée 44. Cette dernière est constituée dans cet exemple par une obstruction totale de la partie postérieure de la glissière 42. En correspondance, une échancrure 45 a été ménagée dans la partie postérieure du coulisseau 41 de sorte qu'une fois ce dernier glissé dans la glissière d'un second dispositif 1, les extrémités postérieures 3 des dispositifs ainsi chaînés se retrouvent toutes alignées dans un même plan.

Afin d'éviter de devoir tenir le dispositif 1 de la main, le moyen d'assemblage 40 peut être pourvu d'une seconde glissière ou d'un second coulisseau 43 situé à l'extrémité postérieure 3, comme bien illustré dans la figure 4. Ce second coulisseau 43 est destiné à venir s'emboîter dans un second moyen d'assemblage complémentaire 53, par exemple une rainure profilée, ménagé dans un support 50 de forme et de dimensions quelconques. Un premier exemple d'un tel support est illustré à la figure 6, laquelle montre également que cette rainure pourrait être réalisée obliquement de manière à ce que le dispositif 1, une fois glissé dans celle-ci, puisse bénéficier d'une position ergonomique favorable pour les manipulations des seringues.

Afin de limiter le déplacement linéaire du deuxième coulisseau par rapport à son moyen d'assemblage complémentaire 53, une seconde butée 54 similaire à la butée 44 pourrait aussi être agencée dans ce support.

Ce support 50 apportera la stabilité nécessaire au dispositif 1 pour que ce dernier puisse tenir debout de lui-même au moyen du second coulisseau 43. Avantageusement, ce support pourra aussi bien accueillir un seul dispositif 1 qu'une pluralité de ces dispositifs par exemple mis en chaîne par le coulisseau 41 et la glissière 42. Selon la forme d'exécution de ce support, il pourrait avantageusement être adapté pour venir se fixer au montant d'un statif de perfusion. En variante, il pourrait être de construction compacte et massive, comme illustré à la figure 6, afin de pouvoir être simplement posé sur un plan ou un plateau de travail. L'ensemble formé de ce support et du ou des dispositifs 1 constitue un présentoir ergonomique dans lequel peuvent être disposées, dans un ordre logique, toutes les seringues nécessaires à l'intervention médicale.

Selon un mode de réalisation possible, le moyen d'assemblage 40 peut être pourvu d'une seconde glissière ou d'un second coulisseau 43, ainsi que d'un coulisseau 41 et d'une glissière 42 disposés aux extrémités latérales 4, 4' du dispositif 1.

Avantageusement, le type d'assemblage par emboîtement du dispositif 1 permet, soit de l'insérer directement au bon endroit dans son support, moyennant un emboîtement élastique ou sans jeu, soit de le glisser et le faire coulisser dans le moyen d'assemblage complémentaire 53, si ce dernier est profilé en queue d'aronde par exemple.

En référence à la figure 4, on remarquera que l'extrémité postérieure 3 du dispositif 1 comprend une troisième ouverture 13 débouchant dans le premier logement 10 en transformant ce dernier en une sorte de canal. Cette ouverture va permettre l'injection de la matière destinée à former l'aiguille 30 lors de l'opération du moulage du dispositif 1. A noter qu'une fois cette opération terminée, cette troisième ouverture 13 pourrait être obturée, au moyen d'un bouchon ou d'un point de soudure par exemple.

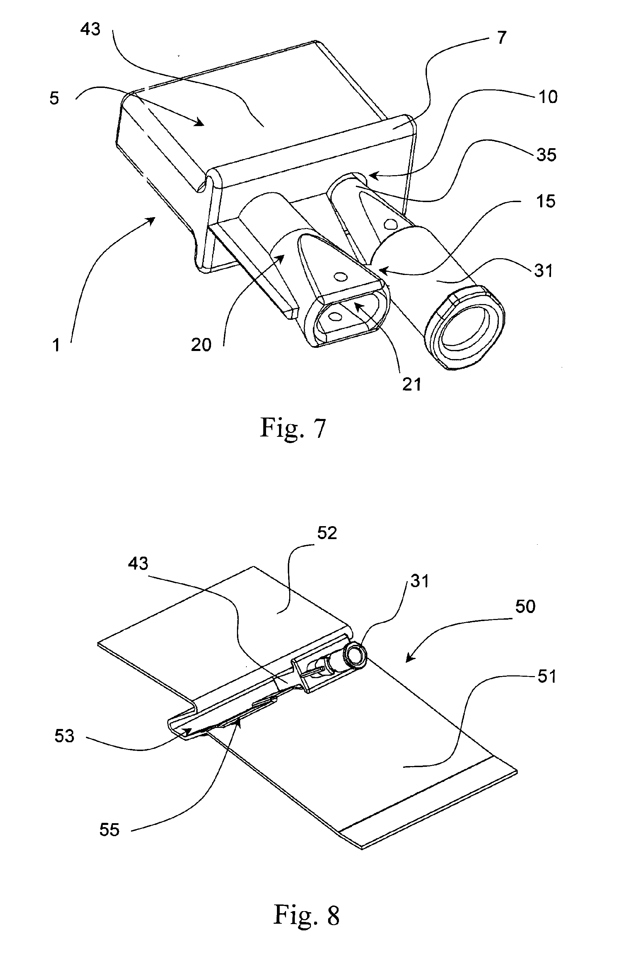

La figure 7 illustre, dans une vue schématique en perspective, une variante du dispositif 1. Ce dernier possède une forme extérieure simplifiée dans laquelle le premier logement 10 et le second logement 20 ne sont apparemment plus distinguables. Ces logements 10, 20 sont dissimulés dans le corps 5 du dispositif 1. De préférence, le logement 10 est séparé du second logement 20 par une cloison à l'intérieur du corps 5. A noter que le nombre de premier et/ou de seconds logements n'est nullement limité à l'unité. De préférence, soit tous les logements 10, 20 sont séparés par un cloisonnement, soit seuls les seconds logements 20 sont séparés les uns des autres par un tel cloisonnement.

En référence à la figure 7, on remarque également que le moyen d'assemblage 40 n'est constitué que du second coulisseau 43, lequel est, dans cet exemple, constitué du corps 5 lui-même.

Dans la partie frontale 2 de ce dispositif 1, se trouve une garde 7 de protection permettant de séparer physiquement la première ouverture 11 et/ou seconde ouverture 21 du corps 5 du dispositif. Plus généralement, on mentionnera qu'au moins un des logements 10, 20 comprend, en cette partie frontale 2, une telle garde de protection. Cette garde a pour premier but de protéger la main du praticien, qui saisira le dispositif 1 par son corps 5, contre toute piqûre intempestive lors des manipulations de ce dispositif avec les seringues; en particulier lors de l'insertion de l'aiguille 30 dans son second logement 20. En outre, cette garde permet également de protéger la zone stérile, que constitue la partie frontale 2 du dispositif, de la zone de préhension que forme le corps 5. De préférence, on remarquera que cette garde 7 s'étend aussi bien du côté supérieur que du côté inférieur du dispositif 1.

Selon la variante du dispositif 1 illustrée à la figure 7, on remarquera également que le moyen de fixation 15 de l'aiguille 30 est constitué par une zone de matière reliant l'embout 31 de l'aiguille 30 à au moins l'un desdits logements 10, 20. De préférence, cette zone de matière comprend une striction, ou zone de rupture, accolée à l'embout 31 pour éviter toute bavure sur la portion conique 35 de l'aiguille après rupture. Ainsi, l'assemblage de cette portion conique 35 dans celle de la seconde ouverture 21 sera toujours parfait lors de introduction de l'aiguille dans son second logement.

Selon le mode de réalisation illustré à la figure 7, la portion conique 35 présente une forme particulière dans laquelle la grande base du cône possède une section droite de forme elliptique alors que la petite base présente une section droite de forme cylindrique. La forme conique de cette portion 35 permet de passer progressivement de la première section à la seconde. Cette forme, que l'on retrouve dans le second logement 20 par le biais du cône de la seconde entrée 21, d'une part permet à l'utilisateur de visualiser facilement dans quelle position il faut insérer l'aiguille, et d'autre part empêche la rotation de l'embout 31 de l'aiguille une fois celle-ci insérée dans le second logement. En utilisant des embouts de type luer-lock, cette forme permet donc de séparer d'une seule main l'aiguille du corps de la seringue en dévissant ce dernier de l'embout 31. La forme en entonnoir de l'entrée 21 facilite également le guidage de cette aiguille.

La figure 8 représente une autre forme d'exécution du support 50, destiné en particulier à la variante du dispositif 1 illustré à la figure 7. Ce support inclut le mode de réalisation préféré d'un moyen d'assemblage complémentaire 53, doté de propriétés élastiques et prévu pour recevoir le moyen d'assemblage 40 du dispositif 1, en particulier le second coulisseau 43 de la variante de ce dispositif. La tenue des dispositifs 1 est assurée par le serrage des parois d'une rainure, laquelle constitue le moyen d'assemblage complémentaire 53, contre le second coulisseau 43 de chaque dispositif 1. L'ouverture de ce moyen d'assemblage complémentaire élastique est obtenue par l'écartement des parois de cette rainure. Pour ce faire, la variante de ce support 50 comprend une patte inférieure 51 et une patte supérieure 52, chacune constituant un prolongement de l'une desdites parois. En agissant sur ces deux pattes 51, 52 comme sur une pincette, on provoque l'ouverture du moyen d'assemblage complémentaire 53 et de ce fait la libération simultanée de tous les dispositifs 1 qui s'y trouvaient insérés.

Avantageusement, la taille généreuse de la patte inférieure 51 permet de stabiliser le support 50 posé sur un plan de travail, dans une position ergonomique facilitant la manipulation des seringues dans les dispositifs 1. Afin de positionner précisément ces dispositifs dans un tel support, il peut être prévu de ménager des logements 55 à l'intérieur du moyen d'assemblage complémentaire 53. De tels logements 55 permettent également de prévenir tout déplacement involontaire des dispositifs 1 lors de l'insertion ou de la reprise d'une seringue. En outre, on remarquera que la garde 7 de la variante du dispositif 1 sert également de butée lorsque ce dispositif est inséré dans le moyen d'assemblage complémentaire 53 d'un tel support. Avantageusement, ce dernier peut être réalisé à moindre frais, par exemple en aluminium ou en acier inox embouti, ou par injection d'une matière plastique conforme aux normes médicales.

Avantageusement encore, la conception du dispositif 1 de la présente invention autorise sa complète réalisation par le biais d'une seule opération de moulage, par injection de matière, préférentiellement par injection d'une matière plastique. Ainsi le premier logement 10, l'aiguille 30 pourvue de son embout 31 et le second logement 20 peuvent avantageusement former un ensemble moulé d'un seul tenant. L'aiguille peut donc être entièrement réalisée en matière plastique, dans le cas où c'est précisément cette matière qui aura été optée pour réaliser l'objet de l'invention. A noter qu'une telle aiguille peut parfaitement être réalisée en une telle matière si elle n'est pas destinée à une injection sous-cutanée mais simplement utilisée pour remplir une seringue, par exemple par percement d'une membrane d'un vial.

Grâce au moyen de fixation 15, chaque aiguille 30 peut aisément être détachée et séparée de son premier logement. Pour ce faire, il suffit de saisir l'aiguille par son embout et de la forcer à se tourner sur son axe principal longitudinal par un mouvement de rotation de la main. La striction ou les cônes de matière qui forment le moyen de fixation 15 cèderont facilement et permettront de libérer l'aiguille, qui de préférence aura préalablement été connectée au corps d'une seringue. On relèvera aussi que la striction ou la forme conique du moyen de fixation 15 permet avantageusement de garantir qu'il casse à l'endroit désiré au moment où l'on cherche à séparer l'aiguille de son premier logement. Bien entendu, les modes de réalisation suggérés ici pour remplir cette fonction ne représentent qu'une conception possible du moyen de fixation 15, lequel pourrait donc être réalisé autrement.

Selon le mode de réalisation préféré, le dispositif 1 n'est pourvu que d'une aiguille 30, d'un seul premier logement 10 et d'un seul second logement 20. En variante, il serait toutefois parfaitement possible de prévoir que le dispositif 1 puisse comprendre une pluralité d'aiguilles 30 abritées soit dans un seul premier logement 10, soit dans autant de premiers logements qu'il y a d'aiguilles. Afin que chaque aiguille usagée puisse être isolée de préférence dans un milieu clos, le nombre de seconds logements 20 sera égal au nombre d'aiguilles 30 que comporte un même dispositif 1.

En raison des multiples rôles qu'il peut remplir par sa fonction de protection des aiguilles stériles, par sa fonction de piège et de bouchon pour les aiguilles usagées et par sa possibilité de pouvoir être assemblé à des dispositifs semblables ou identiques afin de constituer un présentoir ergonomique, on comprendra pourquoi le dispositif 1 de la présente invention a été qualifié de dispositif multifonctionnel.

En outre, ce dispositif pourrait être pré-assemblé au corps d'une seringue et intégré dans son blister afin de limiter les manipulations nécessaires à l'anesthésiste pour utiliser le dispositif.

## Revendications

1. Dispositif (1) multifonctionnel pour aiguilles (30) de seringue comprenant au moins un premier logement (10), abritant au moins une aiguille (30) destinée à en être extraite par un embout (31) constitutif de cette aiguille, et autant de seconds logements (20) qu'il y a d'aiguilles (30) pour piéger ces dernières en les introduisant chacune dans un second logement (20), **caractérisé en ce qu'**au moins le premier logement (10) et ladite aiguille (30), pourvue de son embout (31), forment un ensemble moulé d'un seul tenant.

2. Dispositif (1) selon la revendication 1, **caractérisé en ce que** le premier logement (10), ladite aiguille (30) pourvue de son embout (31) et le second logement (20) forment un ensemble moulé d'un seul tenant.

3. Dispositif (1) selon la revendication 1 ou 2, **caractérisé en ce que** l'aiguille (30) est maintenue dans le premier logement (10) par un moyen de fixation (15) susceptible d'être brisé.

4. Dispositif (1) selon la revendication 3, **caractérisé en ce que** ledit moyen de fixation (15) est constitué d'au moins un cône de matière dont la base est jointe au premier logement (10) et dont le sommet est joint à l'aiguille (30).

5. Dispositif (1) selon la revendication 3, **caractérisé en ce que** ledit moyen de fixation (15) est constitué par une zone de matière reliant l'embout (31) de l'aiguille (30) à au moins l'un desdits logements (10, 20).

6. Dispositif (1) selon la revendication 1 ou 2, **caractérisé en ce qu'**il comprend au moins un moyen d'assemblage (40) par emboîtement permettant une connexion à un moyen d'assemblage complémentaire.

7. Dispositif (1) selon la revendication 6, **caractérisé en ce que** ladite connexion n'autorise qu'un déplacement linéaire desdits moyens d'assemblage l'un par rapport à l'autre une fois emboîtés.

8. Dispositif (1) selon la revendication 7, **caractérisé en ce que** ledit déplacement linéaire est limité par une butée (44).

9. Dispositif (1) selon la revendication 6, **caractérisé en ce que** ledit moyen d'assemblage (40) est pourvu, soit d'au moins un coulisseau (41) et d'au moins une glissière (42), soit d'une seconde glissière ou d'un second coulisseau (43) situé en une extrémité postérieure (3) du dispositif (1), soit d'une seconde glissière ou d'un second coulisseau (43) situé en une extrémité postérieure (3) du dispositif (1) ainsi que d'un coulisseau (41) et d'une glissière (42) disposés en des extrémités latérales (4, 4') du dispositif (1).

10. Dispositif (1) selon la revendication 1, **caractérisé en ce qu'**au moins un desdits logements (10, 20) comprend, en une partie frontale (2), une garde (7) de protection.

11. Dispositif (1) selon la revendication 1, **caractérisé en ce que** ledit ensemble est en matière plastique.

12. Dispositif selon la revendication 1, **caractérisé en ce que** ledit second logement (20) comporte des moyens de positionnement angulaire (26) et des moyens de positionnement en translation (27) de ladite aiguille (30).
